(19) **Europäisches Patentamt**
**European Patent Office**
**Office européen des brevets**

(11) **EP 1 849 779 A1**

(12) **EUROPEAN PATENT APPLICATION**
published in accordance with Art. 158(3) EPC

(43) Date of publication:
**31.10.2007 Bulletin 2007/44**

(21) Application number: **06712293.7**

(22) Date of filing: **25.01.2006**

(51) Int Cl.:
*C07D 311/62* (2006.01)      *A01N 43/16* (2006.01)
*A23L 3/3517* (2006.01)

(86) International application number:
**PCT/JP2006/301098**

(87) International publication number:
**WO 2006/080328 (03.08.2006 Gazette 2006/31)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **26.01.2005   JP  2005017726**

(71) Applicant: **SUNTORY LIMITED**
**Osaka-shi,**
**Osaka 530-8203 (JP)**

(72) Inventors:
• **FUKAMI, Harukazu**
**Kyoto, 6018373 (JP)**

• **NAKAO, Masahiro**
**Kyoto, 6170827 (JP)**
• **NAMIKAWA, Koshi**
**Osaka, 5730092 (JP)**
• **MAEDA, Mitsuru**
**Shiga, 5200244 (JP)**

(74) Representative: **Vossius & Partner**
**Siebertstrasse 4**
**81675 München (DE)**

(54) **ESTERIFIED CATECHIN, PROCESS FOR PRODUCING THE SAME, FOOD AND DRINK OR COSMETIC CONTAINING THE SAME**

(57)    The present invention provides substances having sufficiently antibacterial activity against heat-resistant spore-forming bacteria that they can be used to improve the shelf life of beverages and processed foods, as well as to control the growth of microorganisms in cosmetics.

The present invention provides a catechin ester in which at least one of the hydroxyl groups of a catechin is esterified with a medium-chain fatty acid. This medium-chain fatty acid ester of a catechin, which may be used either alone or as a composition, shows a strong growth suppressing effect on heat-resistant spore-forming bacteria and, if added to foods or beverages, can prevent them from rotting or deterioration and, if added to cosmetics, can prevent their rotting or deterioration.

EP 1 849 779 A1

**Description**

TECHNICAL FIELD

The present invention relates to fatty acid esters of catechins and tea extract that have antibacterial activity to be potentially applicable in foods and beverages, as well as cosmetics.

BACKGROUND ART

In the present day when people have a strong need to feel safe and reassured about foods, it is most important to secure the safety of foods. With growing concern for health, common foods are becoming lower in salt or sugar content but, on the other hand, the water activity of foods has increased to create an environment where microorganisms are prone to propagate. According to one study, at least about 87% of food poisoning cases were of bacterial origin (Yoji Kato, Monthly Food Chemical, August issue, 2001, p. 195). In addition, despite the development of the cold chain, there still is a strong need to secure the preservation and safety of foods. Beverages and the like are sterilized by heat but then heat-resistant spore-forming bacteria can cause deterioration and other undesirable phenomena.

Conventionally, paraoxybenzoic acid, benzoic acid, sorbic acid and the like are used as chemical synthetic preservatives with a view to preventing foods or cosmetics from rotting or deteriorating. In the case where they are added to foods, these compounds are defined as preservatives for food additives and are subject to strict regulations in use. In addition, pectin digests, sardine protein, lysozyme, tea extracts, Hinokitiol and the like are used as shelf-life extenders for food additives. These natural antibacterial agents have such low antibacterial activity that in order to assure complete prevention of deterioration and other undesirable phenomena, they have to be added in large quantities. What is more, many of them contain essential oils as active ingredients and, on account of their characteristic aroma and low solubility in water, the scope and quantity of their use are limited.

Tea extracts are safe and have a suitable degree of solubility in water but, on the other hand, at high enough concentrations that they exhibit a satisfactory antibacterial effect, the tea extracts have the problem of affecting the taste and aroma of the food containing them, as exemplified by the bitterness and astringency of catechin itself as the antibacterial component. In order to solve this problem, various methods have been disclosed, including the combined use of cyclodextrin (JP 3-168046 A) and the use of proteins [the use of egg white, plant proteins, etc. (JP 2-202900 A) or a composition characterized by combined use of defatted egg yolk (JP 2001-31669 A)]. Similarly disclosed are catechins in tea extract that have been transformed to $\alpha$-glycosides with cyclomaltodextrin glucanotransferase with a view to reducing their bitterness or astringency or improving their water solubility (JP 8-298930 A) and the production of $\alpha$-glycosides of catechins with sucrose phosphorylase (JP 5-176786 A). These glycosides are improved in that they have less negative impact on the taste or aroma of foods but on the other hand, no mention is made of the retention of or improvement in antibacterial property. According to JP 11-116418 A, antibacterial substances derived from catechins can be produced by taking special consideration for the temperature and time of heating green tea beverages with a view to providing highly safe but inexpensive naturally occurring antibacterial substances.

Another related prior art is that tea catechins and theaflavins potentiate the antibacterial power of antibiotics against methicillin-resistant Staphylococcus (JP 9-132532 A).

As described above, various studies have been made on the antibacterial property and safety of tea extracts containing catechins as a main ingredient but they are yet to succeed in retaining the antibacterial property of catechins without impairing the taste and aroma as well as color of foods.

Therefore, in order to meet the consumer's demand for higher degrees of safety and reassuredness, antibacterial ingredients are required that have antibacterial activity at lower enough concentrations.

Known fatty acid ester derivatives of catechins include epigallocatechin esterified at the 3-position hydroxyl group by fatty acids (S. Uesato et al., Bioorg. Med. Chem. Lett, 10 (2000) 1673-75, and US Pat. 2003/0105030 A1). These esters are described as having antitumorigenesis promoting activity or 5-$\alpha$ reductase inhibiting activity but no mention is made of their antibacterial activity. In S. Uesato et al. ibid, enzymatic synthesis is performed with carboxylesterase but the substrate for the enzymic reaction involved is an active ester of high reactivity called fatty acid p-nitrophenyl ester and it is not an ordinary fatty acid alkyl ester or triglyceride.

Furthermore, known examples of catechin esterified at the 3-hydroxyl group by fatty acids include decanoyl and palmitoyl esters (JP 54-81274 A). These esters are described as being effective in preventing liver necrosis or hyperoxidation of fat. However, no mention is made of the antibacterial activity of fatty acid ester derivatives of catechins. In addition, no working examples are given for octanoic acid esters of catechin or epicatechin.

Patent Document 1: JP 3-168046 A
Patent Document 2: JP 2-202900 A
Patent Document 3: JP 2001-31669 A
Patent Document 4: JP 8-298930 A

Patent Document 5: JP 5-176786 A
Patent Document 6: JP 11-116418 A
Patent Document 7: JP 9-132532 A
Patent Document 8: JP 54-81274 A
Non-patent Document 1: Kato Yoji, Monthly Food Chemical, August issue, 2001, p. 195
Non-patent Document 2: S. Uesato et al, Bioorg. Med. Chem. Lett, 10(2000) 1673-75
Non-patent Document 3: US P.0105030 A1 (2003)

DISCLOSURE OF THE INVENTION

PROBLEMS TO BE SOLVED BY THE INVENTION

The present invention provides medium-chain fatty acid esters of catechins as substances having antibacterial activity against heat-resistant spore-forming bacteria that are enhanced in the unique antibacterial activity of catechins and which yet are so much reduced in the characteristic bitterness and astringency of catechins that they can be used to improve the shelf life of beverages and processed foods, as well as to control the growth of microorganisms in cosmetics.
The present invention also provides processes for producing the medium-chain fatty acid esters of catechins of the present invention.
The present invention further provides antibacterial agents containing these medium-chain fatty acid esters of catechins as an active ingredient.

MEANS FOR SOLVING THE PROBLEMS

The present inventors conducted intensive studies in which catechins-containing tea extracts or catechins themselves were transformed by the enzymatic or chemical method and an investigation was made to see whether they would be improved in antibacterial activity against heat-resistant spore-forming bacteria. As a result, it was found that by treating catechins with chlorogenate esterase and medium-chain fatty acids, or esters or triglycerides thereof, or by chemically esterifying catechins with medium-chain fatty acids, the original tea extracts or catechins were improved in antibacterial activity against heat-resistant spore-forming bacteria; the present invention has been accomplished on the basis of this finding.
Therefore, the present invention provides a catechin ester in which at least one of the hydroxyl groups of a catechin is esterified with a medium-chain fatty acid (the ester is hereinafter sometimes referred to as a catechin medium-chain fatty acid esters).
The medium-chain fatty acid esters of catechins of the present invention, when eaten or drunk, are readily hydrolyzed with lipase in the digestive tract to return to the original tea extracts or catechins, so they are antibacterial agents of extremely high safety. In other words, the substance of the present invention and the composition containing it have the potential to find utility in such applications as preserving or extending the shelf life of pseudo-drugs, as well as cosmetics and foods or beverages.
In the catechin medium-chain fatty acid ester of the present invention, the ester-forming medium-chain fatty acid preferably contains 8 to 12 carbon atoms, most preferably 8 carbon atoms. The medium-chain fatty acid may be straight or branched and it may be saturated or unsaturated; straight-chained saturated fatty acids are preferred and they include medium-chain fatty acids such as caprylic acid, capric acid and lauric acid. Caprylic acid is most preferred.
Nonlimiting examples of the medium-chain fatty acid ester which are preferred on account of their high antibacterial activity include the following:

a catechin ester in which at least one of the 3-, 5- and 7-hydroxyl groups forms an ester;

a catechin ester in which only the hydroxyl groups at two positions form an ester (simultaneous ester formation at 3'-and 4'-positions may provide somewhat weak antibacterial activity but this is also included within the present invention.)
More specific examples include the following:
a monoester in which a fatty acid forms an ester with the 3-hydroxyl group of a catechin, preferably catechin or epicatechin;
a diester in which a fatty acid forms an ester with the 5-hydroxyl group and 3'- or 4'-hydroxyl group of a catechin, preferably catechin or epicatechin;
a diester in which a fatty acid forms an ester with the 3-hydroxyl group and 3'- or 4'-hydroxyl group of a catechin, preferably catechin or epicatechin; and
a diester in which a fatty acid forms an ester with the 7-hydroxyl group and 3'- or 4'-hydroxyl group of a catechin, preferably catechin or epicatechin.

[Modes of Carrying Out the Invention]

Catechins

Catechins used as the starting material in this specification are preferably catechin, epicatechin, gallocatechin, and epigallocatechin; it is also possible to use catechin gallate, gallocatechin gallate, epicatechin gallate, and epigallocatechin gallate. These compounds have hydroxyl groups and can form esters with medium-chain fatty acids. Part or all of the hydroxyl groups in catechins may be subjected to esterification and other modifications that will not affect their reactivity.

Catechins are contained in tea extracts, so it may be convenient to prepare the starting material for the present invention by using tea extracts as such. Tea extracts are not limited in any particular way and may include: non-fermented teas such as green tea commonly referred to as *sencha*, *hojicha, gyokuro, kabusecha,* and *mushiseicha;* non-fermented teas such as *ureshinocha, aoyagicha,* and a variety of Chinese tea collectively referred to as *kamairicha;* semi-fermented teas such as *houshucha* and oolong tea; fermented teas such as black tea, *awabancha, goishicha,* and Pu-erh tea; and extracts such as maté tea. It is also possible to use commercial tea extracts, such as Sunphenon[®] of Taiyo Kagaku Co., Ltd., Polyphenon[®] of Mitsui Norin Co., Ltd., and SUN OOLONG[®] of SUNTORY LIMITED.

Production by enzymic reaction

To produce catechin medium-chain fatty acid esters from catechins, an enzymic reaction may be used.

Catechins can be used in the reaction as an aqueous liquid. Liquid tea extracts rich in catechins may be used as such. The reaction pH is 3 - 7. Another possible method is such that water is not used in the reaction system but catechins in powder form are added to the reaction system.

Enzymes that can be used in the reaction system include esterases such as chlorogenate esterase and ferulate esterase. These esterases are known as enzymes derived from Asp. japonicus and Lactobacillus acidophilus and are commercially available as enzymes for use in food processing.
For example, it is advantageous to use chlorogenate esterase (Kikkoman Corporation) and ferulate esterase (BIOCON (JAPAN) LTD.)

Using these esterases, hydroxyl groups in catechins can be esterified with medium-chain fatty acids. Medium-chain fatty acids can be used as free acids; alternatively, they may be used in the form of either $C_{1-3}$ alkyl esters of medium-chain fatty acids or triglycerides of medium-chain fatty acids. Medium-chain fatty acids may be subjected to reaction in the presence or absence of water after being mixed with nonpolar solvents such as hexane, benzene and toluene that are inert to the reaction. Under water-free conditions, catechins are added to the reaction system in the form of a powder as described above.

To carry out the reaction, an aqueous solution of catechins and a solution of medium-chain fatty acids, esters or triglycerides thereof, optionally in organic solvents, are first prepared and after adding an enzyme either in powder form or as dissolved in water, the ingredients are left to stand, stirred, shaken or mixed together. The reaction temperature is about 10-60°C, preferably about 30-50°C. The reaction time is sufficient if it is 4-48 hours; if desired, with the oil layer in the reaction solution being analyzed over time, the reaction may be quenched at the point in time when the yield of the ester has reached a maximum. As a guide figure for the relative amounts of catechins, medium-chain fatty acid and enzyme, 1:2-10:0.5-10 (w/w) may be used.

Rather than being used in powder form, the enzyme may be immobilized on a carrier. The enzyme may be immobilized by known methods and the immobilizing carrier may be selected from known carriers such as silica gel, celite, κ-carrageenan, chitin and sodium alginate {Baioriakutah (BIOREACTOR), supervised and compiled by Saburo Fukui, Kodansha-Scientific (1985); Jissen Baioriakutah (Practical Bioreactor), ed. by Shokuhin Sangyo Baioriakutah Shisutemu Gijutsu Kenkyu Kumiai (Technology Study Group on Bioreactor System for Food Industry, Food Chemicals Newspaper Inc. (1990)}. If desired, the enzyme may be used as immobilized on ion-exchange resins of the types used in water treatment. Otherwise, the enzyme may be immobilized on resins that are used in adsorptive chromatography or hydrophobic adsorptive chromatography. The enzyme can also be used as immobilized on resin carriers that can generally adsorb proteins.

If an enzymatic reaction is carried out using catechin or epicatechin as the starting material, the product obtained has the 3-hydroxyl group esterified with the medium-chain fatty acid. The esterified product may be freed of the solvent and used as such; alternatively, it may be purified by silica gel chromatography and/or high-performance liquid chromatography. The esterified product as purified is in solid (powder) form.

Production by chemical synthesis

A catechin such as catechin or epicatechin is reacted either with a medium-chain fatty acid in the presence of a dehydrating/condensing agent such as a carbodiimide derivative or with a medium-chain fatty acid chloride in the presence of a base such as triethylamine or pyridine, in an aprotic solvent or in a solventless manner at 0-60°C for 2-24 hours, whereby there is obtained a mixture of esters ranging from esterification with the fatty acid at one hydroxyl group in the catechin to esterification at all hydroxyl groups. If necessary, the mixture may be purified by usual methods such as silica gel chromatography, optionally followed by high-performance liquid chromatography, in order to separate a fraction that is esterified at the desired position.

The catechins that are esterified at all hydroxyl groups may be treated with alkali metal salts such as sodium hydroxide, sodium carbonate, sodium hydrogencarbonate, potassium hydroxide, potassium carbonate and potassium hydrogencarbonate or organic bases in protic solvents such as water or alcohols at 0-50°C for 2-48 hours, whereby they are converted to catechins that are esterified only at selected hydroxyl groups. Products obtained in this case are primary those which are esterified at the 3-hydroxyl group.

Antibacterial activity

Among the medium-chain fatty acid esters of catechins of the present invention, those in which medium-chain fatty acid is esterified with one or two hydroxyl groups show a strong growth inhibiting action against spore-forming bacteria such as Bacillus or heat-resistant, acidophilic Acidophilus, in comparison with the starting material catechin or epicatechin. Those esters are potentially effective in suppressing the growth of other common bacteria.

The antibacterial activity can be assayed by any suitable known methods, such as the method described in Example 6 below.

EFFECT OF THE INVENTION

The medium-chain fatty acid esters of catechins of the present invention can be used in foods or beverages including green tea, soft drinks, nonalcoholic beverages or alcoholic beverages, or in general foods including seasonings, confectioneries, syrups, processed fruits, processed vegetables, meat products or canned or bottled foods, for the purpose of preserving them or extending their shelf life. They can also be used in the cosmetic field for the purpose of preventing putrefaction or deterioration of cosmetic cream, ointments or cosmetic liquids.

EXAMPLES

On the following pages, the present invention is described more specifically with reference to examples which are by no means intended to limit the scope of the present invention.

Example 1 Chemical Synthesis of Catechin Esters

Catechin hydrate (3.05 g, 10.5 mmol) was dissolved in 20 ml of tetrahydrofuran (THF) and 2.5 ml (31.1 mmol) of pyridine was added. The liquid mixture was cooled on ice and 3.6 ml (21.1 mmol) of caprylic acid chloride was added. After the addition, the mixture was stirred overnight at room temperature. To the reaction solution, ethyl acetate (AcOEt) was added, washed successively with a saturated aqueous solution of potassium hydrogensulfate, water, saturated aqueous sodium hydrogencarbonate, and saturated aqueous sodium chloride, dried with anhydrous sodium sulfate, followed by distilling off the solvent under vacuum. The resulting residue was fractionated by silica gel chromatography (AcOEt/hexane = 1/2 - 2/1). Among the diester fractions, 3'4'-O-dioctanoylcatechin was obtained as a high-purity fraction in 1.25 g by TLC (AcOEt/hexane = 1:1). In addition, high-purity 3'(and 4')-O-octanoylcatechin was obtained in an amount of 0.78 g. The 3'- and 4'-bound octanoyl groups transferred to each other to form a near 1:1 mixture.

3'4'-O-dioctanoyl-(+)-D-catechin: White crystal, $^1$H-MMR (DMSO-d6, ppm): 0.87 (6H, m), 1.2-1.4 (16H, m), 1.60 (4H, m), 2.39 (1H, dd), 2.55 (4H, t), 2.75 (1H, dd), 3.86 (1H, m), 4.65 (1H, d), 5.11 (1H, d), 5.72 (1H, d), 6.00 (1H, d), 7.2-7.4 (3H, m), 8.99 (1H, s), 9.24 (1H, s).

3'-O-cotanoyl and 4'-octanoyl-(+)-D-catechin: Amorphous, $^1$H-MMR (DMSO-d6, ppm): 0.87 (3H, t), 1.2-1.4 (8H, m), 2.16 (2H, m), 1.63 (2H, m), 2.37 (1H, m), 2.54 (2H, t), 2.69 (1H, m), 3.83 (1H, m), 4.55 (1H, dd), 5.00 (1H, dd), 5.70 (1H, d), 5.90 (1H, s), 6.7-7.1 (3H, m), 9.05 (1H, s), 9.20 (1H, s), 9.59 (1H, s).

Example 2 Chemical Synthesis of Catechin Esters

Catechin hydrate (3.04 g, 10.5 mmol) was dissolved in 20 ml of THF and 3.8 ml (47.2 mmol) of pyridine was added. The liquid mixture was cooled on ice and 5.4 ml (31.6 mmol) of caprylic acid chloride was added. After the addition, the mixture was stirred overnight at room temperature. To the reaction solution, AcOEt was added, washed successively with a saturated aqueous solution of potassium hydrogensulfate, water, saturated aqueous sodium hydrogencarbonate, and saturated aqueous sodium chloride, dried with anhydrous sodium sulfate, followed by distilling off the solvent under vacuum. The resulting residue was fractionated by silica gel chromatography (AcOEt/hexane = 1/2 - 2/1). Among the diester fractions, 3'(or 4'),5-di-O-octanoylcatechin and 3'(or 4'),3-di-O-octanoylcatechin were obtained as high-purity fractions in 0.17 g and 0.10 g, respectively, by TLC (AcOEt/hexane = 1:1). Another fraction obtained (0.54 g) consisted of two components and a portion of it was purified by HPLC (column: Develosil C30-UG-5, 10 x 250 mm, eluting solution: 80% acetonitrile in water) to give 3'(or 4'),7-di-O-octanoylcatechin and 3'4'-O-dioctanoylcatechin.

3'(or 4'),5-di-O-octanoyl-(+)-D-catechin: $^1$H-NMR (DMSO-d6, ppm): 0.85 (6H, m), 1.2-1.4 (16H, m), 1.63 (4H, m), 2.18 (1H, m), 2.36 (1H, m), 2.54 (4H, m), 3.86 (1H, m), 4.63 (1H, dd), 5.13 (1H, dd), 6.0-6.2 (2H, m), 6.7-7.1 (3H, m), 9.5-9.7 (2H, m).

3'(or 4'),3-di-O-octanoyl-(+)-D-catechin: $^1$H-NMR (DMSO-d6, ppm): 0.86 (6H, m), 1.1-1.4 (18H, m), 1.62 (2H, m), 2.15 (2H, m), 2.70 (1H, m), 4.9-5.2 (2H, m), 5.78 (1H, d), 5.94 (1H, s), 6.7-7.0 (3H, m), 9.08 (1H, d), 9.36 (1H, s), 9.69 (1H, s).

3'(or 4'),7-di-O-octanoyl-(+)-D-catechin: $^1$H-NMR (DMSO-d6, ppm): 0.86 (6H, m), 1.2-1.4 (16H, m), 1.60 (4H, m), 2.38 (1H, m), 2.55 (4H, t), 2.75 (1H, m), 3.90 (1H, m), 4.6-4.8 (1H, m), 5.0-5.2 (1H, m), 5.7-6.2 (2H, m), 6.7-7.4 (3H, m), 8.9-9.8 (2H, m).

Example 3 Chemical Synthesis of Catechin Esters

Catechin hydrate (3 g, 10.3 mmol) was dissolved in 15 ml of THF and 6.3 ml (78.3 mmol) of pyridine was added. The liquid mixture was cooled on ice and 9.7 ml (56.8 mmol) of caprylic acid chloride was added. After the addition, the mixture was stirred overnight at room temperature. To the reaction solution, AcOEt was added, washed successively with a saturated aqueous solution of potassium hydrogensulfate, water, saturated aqueous sodium hydrogencarbonate, and saturated aqueous sodium chloride, dried with anhydrous sodium sulfate, followed by distilling off the solvent under vacuum. The resulting residue was fractionated by silica gel chromatography (AcOEt/hexane = 1/20 - 1/4). Upon concentrating under vacuum, penta-O-octanoylcatechin was obtained in 7.0 g and 5,7,3',4'-tetra-O-octanoylcatechin in 1.3 g. The penta-O-octanoylcatechin (7.0 g, 7.6 mmol) was dissolved in 20 ml of methylene chloride and after adding ethanolamine (1.74 ml, 28.8 mmol), the mixture was stirred overnight at room temperature. The reaction solution was diluted with AcOEt/hexane = 1/4 and fractionated as such by silica gel chromatography (AcOEt/hexane = 1/4 - 2/1). The diester fraction was purified by a second run of chromatography (methylene chloride/acetone = 10/1 - 4/1) to obtain high-purity 3,7-di-O-octanoylcatechin (75 mg). In addition, the monoester fraction was concentrated to obtain 1.46 g of 3-O-otanoylcatechin.

3,7-di-O-octanoyl-(+)-D-catechin: $^1$H-NMR (DMSO-d6, ppm): 0.85 (6H, m), 1.1-1.4 (18H, m), 1.59 (2H, m), 2.15 (2H, t), 2.4-2.6 (4H, m), 5.00 (1H, d), 5.13 (1H, q), 6.13 (1H, d), 6.21 (1H, d), 6.56 (2H, dd), 6.6-6.7 (2H, m), 8.95 (2H, s), 9.60 (1H, s).

3-O-cotanoyl-(+)-D-catechin: Amorphous, $^1$H-MMR (DMSO-d6, ppm): 0.85 (3H, t), 1.1-1.5 (10H, m), 2.16 (2H, m), 2.64 (1H, dd), 4.90 (1H, dd), 5.10 (1H, q), 5.77 (1H, d), 5.93 (1H, d), 6.57 (1H, dd), 6.7-6.8 (2H, m), 8.91 (2H, bs), 9.05 (1H, s), 9.32 (1H, s).

Example 4 Chemical Synthesis of Catechin Ester

Penta-O-octanoylcatechin (6.6 g, 7.2 mmol) was dissolved in 20 ml of methylene chloride and after adding ethanolamine (1.21 ml, 20.0 mmol), the mixture was stirred overnight at room temperature. The reaction solution was diluted with AcOEt/hexane = 1/4 and fractionated as such by silica gel chromatography (AcOEt/hexane = 1/4 - 2/1). Among the diester fractions, 3,5-di-O-octanoylcatechin (180 mg) was obtained as a high-purity fraction on TLC.

3,5-di-O-octanoyl-(+)-D-catechin: 0.85 (6H, m), 1.0-1.4 (16H, m), 1.48 (2H, t), 1.61 (2H, t), 2.19 (2H, t), 2.5-2.7 (4H, m), 4.9-5.3 (2H, m), 6.11 (1H, d), 6.15 (1H, d), 6.57 (1H, dd), 6.6-6.8 (2H, m), 9.06 (2H, brs), 9.86 (1H, brs).

Example 5 Chemical Synthesis of Catechin Ester

Tetra-O-octanoylcatechin (3.5 g, 4.4 mmol) was dissolved in 10 ml of methylene chloride and after adding ethanolamine (0.47 ml, 7.8 mmol), the mixture was stirred overnight at room temperature. The reaction solution was diluted with AcOEt/hexane = 1/4 and fractionated as such by silica gel chromatography (AcOEt/hexane = 1/4 - 2/1). As a diester fraction, high-purity 5,7-di-O-octanoylcatechin (0.3 g) was obtained on TLC.

5,7-di-O-octanoyl-(+)-D-catechin: $^1$H-NMR (DMSO-d6, ppm): 0.87 (6H, m), 1.2-1.4 (16H, m), 1.6-1.7 (4H, m), 2.4-2.7 (4H, m), 3.93 (1H, q), 4.71 (1H, d), 6.51 (2H, dd), 6.59 (1H, dd), 6.6-6.8 (2H, m), 8.90 (2H, brs).

Example 6 Assay of Antibacterial Activity:

The substances of the present invention that were prepared in Examples 1-5 and the catechin prepared in Reference Example 1 to be described later were dissolved in dimethyl sulfoxide at various concentrations and 2 $\mu$l-portions of the respective samples were placed on flat-bottomed 96-well multi-plates (Corning Coster); thereafter, 50 $\mu$l of a physiological saline suspension of Bacillus subtilis spores (1 x $10^7$/ml) was added; the spores had been prepared in a spore forming medium (polypeptone, 10.0 g; yeast extract, 5.0 g; calcium carbonate, 15.0 g; agar, 15.0 g; an aqueous solution of inorganic salts, 5.0 ml; distilled water, 1000 ml; provided that the aqueous solution of inorganic salts consisted of 4.0 g of magnesium sulfate heptahydrate, 0.216 g of manganese(II) sulfate pentahydrate, 0.2 g of iron(II) sulfate heptahydrate, 0.2 g of sodium chloride, and 100 ml of distilled water). Into each well, 50 $\mu$l of a nutrient broth (pH 7.0, NISSUI PHARMACEUTICAL CO., LTD.) was added and cultured overnight at 37°C. The light absorbance at 690 nm was measured both at the start and end of the culture by means of a micro-plate reader (Thermolab Systems, Finland). Based on the percent growth inhibition calculated from the following formula, a distribution diagram was constructed plotting percent inhibition and sample concentration on the two axes and the 50% inhibition (IC50) of the spore forming bacterium by the substances of the present invention was determined.
**Percent growth inhibition (%) =**

**100 x {Δ690 (not added) - Δ690 (sample added)}/Δ690 (not added)**

where Δ690 (not added) refers to the difference between the 690 nm values in the "not-added" group before and after the culture and Δ690 (sample added) refers to the difference between the 690 nm values in the "sample-added" group before and after the culture. The results are shown in Table 1.
Activity against the heat-resistant acidophilic spore-forming bacterium Alicyclobacillus acidoterrestris was determined by taking the same procedure, except that a change was made in two points, the culture temperature condition and the type of liquid medium [culture temperature: 45°C; liquid medium: 0.4% dry yeast extract (Difco Laboratories), 0.4% soluble starch (nacalai tesque), 1% glucose (nacalai tesque), adjusted to pH 3.8 with 1 N sulfuric acid].
The results are shown in Table 1.
Table 1

|  | IC50 (ppm) | |
| --- | --- | --- |
|  | B. subtilis | A. acidoterrestris |
| 3'-O-cotanoyl and 4'-octanoyl-(+)-D-catechin | 200-400 | 25-50 |
| 3'-O-cotanoyl-(+)-D-catechin | 12.5-25 | 12.5-25 |
| 3'4'-O-dioctanoyl-(+)-D-catechin | >400 | 12.5-25 |
| 3'(or 4'),5-di-O-octanoyl-(+)-D-catechin | 6.25-12.5 | 6.25-12.5 |
| 3'(or 4'),3-di-O-octanoyl-(+)-D-catechin | 3.125-6.25 | 6.25-12.5 |
| 3'(or 4'),7-di-O-octanoyl-(+)-D-catechin | 6.25-12.5 | 50-100 |
| 3,7-di-O-octanoyl-(+)-D-catechin | 25-50 | 12.5-25 |
| 5,7-di-O-octanoyl-(+)-D-catechin | >400 | 100-200 |
| 3,5-di-O-octanoyl-(+)-D-catechin | 100-200 | 50-100 |
| Sucrose fatty acid ester | 50-100 | 100-200 |
| 3-O-decanoyl-(+)-catechin | 6.25-12.5 | 10 |
| Catechin | >400 | 50-100 |

Example 7 3-O-Octanoyl-(+)-D-Catechin (Enzymatic Synthesis)

Search was made through commercial lipase and esterase enzyme preparations by referring to the retention time of 14.2 minutes for a chemical synthetic product of 3-O-capryloylcatechin on an HPLC system (HITACHI Model D-7000, Diode-Array Detector Model L-7451) and a column Develosil C30-UG-5 (NOMURA CHEMICAL CO, LTD., 4.6 x 150 mm; mobile phase: 5%-90% acetonitrile/0.1% TFA gradient elution in 0-15 min; flow rate: 1.0 ml/min; detection wavelength: 230 nm or 280 nm). The enzymic reaction system was prepared as follows: 5 mg of catechin was dissolved in 0.3 ml of 20 mM acetate buffer solution (pH 5.0) and, thereafter, each enzyme preparation was added and following the addition of 0.3 ml of octanoic acid, the mixture was shaken for reaction at 37°C. The oil layer portion of the reaction solution was analyzed by HPLC to reveal that chlorogenate esterase (Kikkoman Corporation) had the activity for ester transfer to catechin. A peak was observed at the 14.2-min position which immediately followed the octanoic acid peak (12.9 min); the analysis by co-chromatography with the chemical synthetic product showed an agreement of retention time to the 3-O-capryloylcatechin.

Example 8 3-O-Octanoyl-(+)-D-Catechin (Enzymatic Synthesis)

Catechin (5 g) was dissolved in 300 ml of buffer; to the solution, 50 g of chlorogenate esterase and 300 ml of octanoic acid were added and the mixture was stirred with a stirrer while the reaction was carried out for 20 hours. The reaction solution was separated into two layers; the oil layer was washed with water and distilled under vacuum (120°C, 2 mmHg) to remove octanoic acid; then, the distillation residue was partially purified by silica gel chromatography (eluted by methylene chloride and methylene chloride/methanol (1%-30% gradient) to give 1.3 g of a crude product. Part of this crude product was separated using the preparative column Develosil ODS-HG-5 (NOMURA CHEMICAL CO, LTD., 10 x 250 mm; mobile phase: 70% acetonitrile/0.1% TFA; flow rate: 1.3 ml/min; 1.3 ml fractionated) to obtain a high-purity sample of the titled compound. Its NMR and mass spectra agreed with those of the corresponding synthetic product.

Example 9 Enzymatic Synthesis of 3-O-Octanoyl-(+)-D-Epicatechin

Epicatechin (5 g) was dissolved in 300 ml of buffer; to the solution, 50 g of chlorogenate esterase and 300 ml of ethyl octanoate were added and the mixture was stirred with a stirrer while the reaction was carried out for 20 hours. The reaction solution was separated into two layers; the oil layer was washed with water, dried with anhydrous sodium sulfate, and partially purified by silica gel chromatography (ethyl caprylate was first eluted with methylene chloride, then with methylene chloride/methanol (1%-30% gradient)) to give 1.1 g of a crude product. Part of this crude product was separated using the preparative column Develosil C30-UG-5 (NOMURA CHEMICAL CO, LTD., 10 x 250 mm; mobile phase: 60% acetonitrile/0.1% TFA; flow rate: 1.3 ml/min; 0.65 ml fractionated) to obtain a high-purity sample of the titled compound.

Example 10 Enzymatic Synthesis of Esters Using Various Fatty Acid Donors

Octanoic acid (C8), ethyl octanoate (C8Et) and octanoic acid triglyceride (MCT) as fatty acid donors were subjected to enzymic reaction as in Example 8 and the oil layer portion of each reaction solution was analyzed by HPLC over time; increased ester peaks were recognized for the respective fatty acid donors.
Table 2

| | Ester yield (mg/ml) | |
| --- | --- | --- |
| | 5 h | 24 h |
| C + C8 | 0.26 | 0.31 |
| EC + C8 | 0.12 | 0.23 |
| C + C8Et | 0.20 | 0.60 |
| EC + C8Et | 0.17 | 0.31 |
| C + MCT | 1.22 | 1.42 |

(continued)

| Ester yield (mg/ml) | | |
|---|---|---|
| EC + MCT | 0.99 | 0.79 |

C: catechin; EC: epicatechin; C8: octanoic acid; C8Et: ethyl octanoate; MCT: octanoic acid triglyceride

The above results ascertained that in the enzymatic method according to the present invention, not only free octanoic acid but also ethyl octanoate and octanoic acid triglyceride served as fatty acid donors to generate the corresponding fatty acid esters of catechin and epicatechin.

Reference Example 1 Synthesis of 3-O-Decanoyl-(+)-Catechin

As in Example 4, crude penta-O-decanoylcatechin (23.5 g) was obtained from catechin hydrate (6.3 g, 21.8 mmol) and caproic acid chloride (25 g, 131.1 mmol). A 6-g portion of the crude product was dissolved in 20 ml of methylene chloride and after adding ethanolamine (1.75 ml, 29.0 mmol), the mixture was stirred overnight at room temperature. The reaction solution was diluted with AcOEt/hexane = 1/4 and fractionated as such by silica gel chromatography (AcOEt/hexane = 1/4 - 2/1). The resulting monoester fraction was concentrated to obtain 1.25 g of 3- O-decanoylcatechin (amorphous).
$^1$H-NMR (DMSO-d6, ppm): 0.85 (3H, t), 1.1-1.5 (12H, m), 2.1-2.2 (2H, m), 2.6-2.7 (1H, m), 4.90 (1H, d), 5.10 (1H, q), 5.76 (1H, d), 5.92 (1H, d), 6.56 (1H, dd), 6.6-6.8 (2H, m), 8.88 (1H, s), 8.93 (1H, s), 9.05 (1H, s), 9.32 (1H, s).

## Claims

1. An antibacterial agent containing at least one catechin ester as an active ingredient, the ester having at least one of the hydroxyl groups of a catechin esterified with a medium-chain fatty acid.

2. The antibacterial agent according to claim 1, wherein the ester-forming medium-chain fatty acid contains 8 to 12 carbon atoms.

3. The antibacterial agent according to claim 2, wherein the ester-forming medium-chain fatty acid contains 8 carbon atoms.

4. The antibacterial agent according to any one of claims 1 to 3, wherein at least the 3-hydroxyl group of the catechin forms an ester.

5. The antibacterial agent according to any one of claims 1 to 3, wherein at least the 5-hydroxyl group of the catechin forms an ester.

6. The antibacterial agent according to any one of claims 1 to 3, wherein at least the 7-hydroxyl group of the catechin forms an ester.

7. The antibacterial agent according to any one of claims 4 to 6, wherein the hydroxyl groups at two positions form an ester.

8. The antibacterial agent according to any one of claims 1 to 3, which contains at least one catechin ester as an active ingredient that is selected from the group consisting of:

   a monoester in which the fatty acid forms an ester with the 3-hydroxyl group of the catechin;
   a diester in which the fatty acid forms an ester with the 5-hydroxyl group and 3'- or 4'-hydroxyl group of the catechin;
   a diester in which the fatty acid forms an ester with the 3-hydroxyl group and 3'- or 4'-hydroxyl group of the catechin; and

a diester in which the fatty acid forms an ester with the 7-hydroxyl group and 3'- or 4'-hydroxyl group of the catechin.

9.  The antibacterial agent according to any one of claims 1 to 8, which is for use in foods, beverages, cosmetics or medicaments.

10. The antibacterial agent according to any one of claims 1 to 9, which has antibacterial activity against heat-resistant spore-forming bacteria.

11. A process for producing a catechin ester, in which a catechin or an ester derivative thereof undergoes ester formation by means of chlorogenate esterase or ferulate esterase or is subjected to transesterification reaction, both in the presence of a fatty acid donor selected from the group consisting of medium-chain fatty acids, esters or triglycerides thereof, whereby at least one of the hydroxyl groups in the catechin is esterified with the medium-chain fatty acid.

12. The process according to claim 11, wherein the ester formation or transesterification reaction is performed in a buffer solution of pH 3-7 at a temperature of 30-50°C for a period of 4-48 hours.

13. The process according to claim 11 or 12, wherein the fatty acid donor is octanoic acid, ethyl octanoate or octanoic acid triglyceride.

14. The process according to any one of claims 11 to 13, wherein the medium-chain fatty acid forms an ester at the 3-position of catechin.

15. The process according to any one of claims 11 to 14, wherein the catechin is used in the reaction in the form of a tea extract.

16. A catechin ester in which at least one of the hydroxyl groups in a catechin is esterified with a medium-chain fatty acid, provided that the catechin ester is not 3-0-decanoylcatechin.

17. The catechin ester according to claim 16, wherein the ester-forming medium-chain fatty acid contains 8 to 12 carbon atoms.

18. The catechin ester according to claim 17, wherein the ester-forming medium-chain fatty acid contains 8 carbon atoms.

19. The catechin ester according to any one of claims 16 to 18, wherein at least the 3-hydroxyl group of the catechin forms an ester.

20. The catechin ester according to any one of claims 16 to 18, wherein at least the 5-hydroxyl group of the catechin forms an ester.

21. The catechin ester according to any one of claims 16 to 18, wherein at least the 7-hydroxyl group of the catechin forms an ester.

22. The catechin ester according to any one of claims 19 to 21, wherein the hydroxyl groups at two positions form an ester.

23. The catechin ester according to any one of claims 16 to 18, which is selected from the group consisting of:

    a monoester in which the fatty acid forms an ester with the 3-hydroxyl group of the catechin;
    a diester in which the fatty acid forms an ester with the 5-hydroxyl group and 3'- or 4'-hydroxyl group of the catechin;
    a diester in which the fatty acid forms an ester with the 3-hydroxyl group and 3'- or 4'-hydroxyl group of the catechin; and
    a diester in which the fatty acid forms an ester with the 7-hydroxyl group and 3'- or 4'-hydroxyl group of the catechin.

## INTERNATIONAL SEARCH REPORT

International application No.

PCT/JP2006/301098

### A. CLASSIFICATION OF SUBJECT MATTER
*C07D311/62*(2006.01), *A01N43/16*(2006.01), *A23L3/3517*(2006.01),
*A61K8/49*(2006.01), *A61K31/353*(2006.01), *A61K47/22*(2006.01),
*C12P17/06*(2006.01)

According to International Patent Classification (IPC) or to both national classification and IPC

### B. FIELDS SEARCHED

Minimum documentation searched (classification system followed by classification symbols)
*C07D311/62*(2006.01), *A01N43/16*(2006.01), *A23L3/3517*(2006.01),
*A61K8/49*(2006.01), *A61K31/353*(2006.01), *A61K47/22*(2006.01),
*C12P17/06*(2006.01)

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN)

### C. DOCUMENTS CONSIDERED TO BE RELEVANT

| Category* | Citation of document, with indication, where appropriate, of the relevant passages | Relevant to claim No. |
|---|---|---|
| X<br>A | Ki Duk PARK, et.al., Antimicrobial Activity of 3-O-Acyl-(-)-epicatechin and 3-O-Acyl-(+)-catechin derivatives., Planta Med., 2004, Vol.70, No. 3, pages 272-276 | 1-4,8-9,<br>16-19,23<br>5-7,10-15,<br>20-22 |
| X<br>A | Shinichi UESATO, et al., Inhibitory Effects of 3-O-Acyl-(+)-catechins on Epstein-Barr Virus Activation., Chem.Pharm.Bull., 2003, Vol.51, No.12, pages 1448-1450 | 16-19,23<br>1-15,20-22 |
| X<br>A | JP 2003-524577 A (Arch Development Corp.), 19 August, 2003 (19.08.03), Claim 10; Fig. 5<br>& WO 99/022728 A1 & EP 1534298 A2<br>& CN 1357003 A | 16-19,23<br>1-15,20-22 |

☒ Further documents are listed in the continuation of Box C.   ☐ See patent family annex.

| | |
|---|---|
| \*   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search<br>  20 March, 2006 (20.03.06) | Date of mailing of the international search report<br>  04 April, 2006 (04.04.06) |
|---|---|
| Name and mailing address of the ISA/<br>  Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/301098

C (Continuation). DOCUMENTS CONSIDERED TO BE RELEVANT

| Category* | Citation of document, with indication, where appropriate, of the relevant passages | Relevant to claim No. |
|---|---|---|
| A | JP 6-279430 A (Mitsui Norin Co., Ltd.), 04 October, 1994 (04.10.94), Claims 1, 2; Par. No. [0004] & EP 618203 A1 | 1-23 |
| A | JP 8-259555 A (Kawaken Fine Chemicals Co., Ltd.), 08 October, 1996 (08.10.96), Claims 1, 2 (Family: none) | 1-23 |
| A | SCEVOLA D., et. al., Enhancement of Chemotaxis and Phagocytosis by Flavonoid 3-Palmitoyl-(+)-Catechin in Galactosamine Hepatitis., INT. J. IMMUNOTHERAPY, 1988, Vol.IV, No.1, pages 41-47 | 1-23 |
| A | US 5844061 A (Berkem), 01 December, 1998 (01.12.98), & WO 94/029404 A1          & FR 2706478 A | 1-23 |

Form PCT/ISA/210 (continuation of second sheet) (April 2005)

EP 1 849 779 A1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3168046 A
- JP 2202900 A
- JP 2001031669 A
- JP 8298930 A
- JP 5176786 A
- JP 11116418 A
- JP 9132532 A
- US 20030105030 A1
- JP 54081274 A
- US 0105030 A1

**Non-patent literature cited in the description**

- **YOJI KATO.** Monthly Food Chemical. August 2001, 195
- **S. UESATO et al.** *Bioorg. Med. Chem. Lett,* 2000, vol. 10, 1673-75
- **KATO YOJI.** *Monthly Food Chemical,* August 2001, 195